# EUROPEAN PATENT APPLICATION

(11) **EP 4 772 628 A1**
(43) Date of publication of application: **08.07.2026**
(21) Application number: 23955859.6
(22) Date of filing: 31.10.2023
(51) Int. Cl.: C12N 15/11, C12N 15/10, C12N 9/00

(54) **PREPARATION METHOD FOR NON-NATURAL RNA AND PRODUCT**

(30) Priority: 16.10.2023 CN 202311335114
(71) Applicant: Jilin Asymchem Laboratories Co., Ltd., Dunhua, Jilin 133700 (CN); Tianjin Asymchem Pharmaceuticals Co., Ltd, Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); LIU, Fang, Tianjin 300457 (CN); MA, Cuiping, Tianjin 300457 (CN); PANG, Huining, Tianjin 300457 (CN); JIA, Xu, Tianjin 300457 (CN); WANG, Zhaoshuai, Tianjin 300457 (CN); GENG, Yuhan, Tianjin 300457 (CN); CUI, Lixin, Tianjin 300457 (CN); ZHU, Wenxuan, Tianjin 300457 (CN); ZHAO, Xiaolan, Tianjin 300457 (CN)
(74) Representative: HGF
(86) International application number: PCT/CN2023/128828
(87) International publication number: WO 2025/081541

(57) **Abstract**

Provided is a method for preparing non-natural RNA, and a product. The above method for preparing non-natural RNA includes: linking the 3' end of a substrate donor with the 5' end of a substrate receptor to form a phosphodiester bond with a RNA ligase, where a linkage does not require a template strand to prepare non-natural RNA; the substrate donor and/or the substrate receptor includes one or more non-natural nucleotides; the 5' end of the substrate receptor is a phosphate group; the 3' end of the substrate donor is a hydroxyl group; and the RNA ligase includes any one or more enzymes in the RNA ligase family Rnl1 and Rnl2. Therefore, the problem in the prior art of difficult preparation of non-natural RNA using enzyme catalysis is solved, and the method is suitable for the field of RNA synthesis.

## Description

This application is based upon and claims priority to Chinese Patent Application No. 202311335114.7 filed on October 16, 2023, the disclosure of which is hereby incorporated by reference in its entirety.

### Technical Field

The present disclosure relates to the field of RNA synthesis, and specifically, to a method for preparing non-natural RNA, and a product.

### Background

RNA is the carrier of genetic information found in biological cells and certain viruses and viroid. For a long time, the RNA was considered merely a molecule that transmits information between genes and proteins. At the dawn of life, the RNA was the sole life molecule capable of both storing information and achieving enzymatic functions. The RNA not only serves as the messenger for protein synthesis (mRNA), but also has crucial regulatory functions. Non-transcription RNA includes miRNA, siRNA, IncRNA, piwiRNA, etc. There are over 400 miRNA molecules, regulating at least one-third of human genes. Since the 1970s, the gene vector technology, gene cloning technology, and gene editing technology, etc. have profoundly impacted modern gene therapy techniques. Moreover, the RNA editing technology enables the editing of specific nucleotides in human cells at the genetic level. The technology may serve not only as a research tool, but also as a temporary therapeutic method for diseases caused by mutations.

In recent years, the field of RNA drugs has developed rapidly. On one hand, small nucleic acid drugs represented by siRNA and ASO have made significant strides in fields such as rare diseases and the like. On the other hand, mRNA drugs represented by mRNA vaccines have played a crucial role in the COVID-19 pandemic. Currently, the most common synthesis method for oligonucleotides (12-30 nucleotides) is solid-phase synthesis. Solid-phase synthesis includes fixing nucleic acids onto a solid-phase carrier to complete a synthesis reaction. The most commonly used solid-phase carrier is Controlled-Pore Glass (CPG) beads, but is limited in loading capacity, typically ranging from 70-80 µmol/g. However, solid-phase synthesis of nucleotide chains has significant drawbacks. The initial oligonucleotide products are low in purity, limited in chain length, and typically suitable for synthesizing chains up to 25 nucleotides. As the synthesized chain length increases, yield decreases; and when primers are synthesized to form a chain with the length being about 80 nt, the purity of the crude product is only about 40%. Such low purity simultaneously impacts purification yields, resulting in low purification rates, and thus severely limiting nucleotide chain synthesis. Moreover, the substantial consumption of organic reagents exacerbates the difficulty of treating the three wastes and causes significant environmental pollution.

Natural oligonucleotides are prone to degradation in vivo, low in specificity, and have toxic side effects. Therefore, drug oligonucleotides are typically subjected to specific chemical modifications to improve their stability in vivo, improve specificity, and reduce toxicity. These chemical modifications include fluorination, methylation, and methoxyethyl modifications. Compared to traditional drugs that act at the protein level, nucleic acid drugs have significant advantages such as high specificity, high efficacy, long-lasting effects, etc. However, since natural nucleic acids are very sensitive to both endonucleases and exonucleases and highly susceptible to degradation, nucleic acid drugs use chemical modifications to improve stability, reduce immunogenicity, and improve specificity. Simply-chemically modified nucleic acids are artificially created and not natural. Therefore, the activity of natural enzymes toward non-natural nucleic acids remains unknown.

Enzyme-catalyzed synthesis is a green and highly efficient manner, which is characterized by mild reaction conditions and minimal or no requirement for organic reagents, and thus is a promising catalytic method. Using ligases to synthesize nucleotide chains may realize green catalysis, while the synthesized chain length is not limited, such that technical bottlenecks in solid-phase synthesis can be solved. However, the application of the ligases to link single-stranded RNA is largely confined to natural RNA strands, and there are virtually no reports in the literature or patents regarding the enzymatic synthesis of non-natural single-stranded RNA.

### Summary

The present disclosure is mainly intended to provide a method for preparing non-natural RNA, and a product, so as to solve the problem in the prior art of difficult preparation of non-natural RNA using enzyme catalysis.

In order to implement the above objective, according to a first aspect of the present disclosure, a method for preparing non-natural RNA is provided. The preparation method includes: the 3' end of a substrate donor is linked with the 5' end of a substrate receptor to form a phosphodiester bond by using a RNA ligase, where the linking does not require a template strand to prepare the non-natural RNA; the substrate donor and/or the substrate receptor includes one or more non-natural nucleotides; the 5' end of the substrate receptor is a phosphate group; the 3' end of the substrate donor is a hydroxyl group; and the RNA ligase includes any one or more enzymes in the RNA ligase family Rnl1 and Rnl2.

Further, the RNA ligase includes one or more of the RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 22; or an enzyme that has more than 70% identity to any one of the RNA ligases shown in SEQ ID NO: 1-SEQ ID NO: 8 or SEQ ID NO: 15-SEQ ID NO: 22, and has an activity for catalyzing the formation of the phosphatediester bond.

Further, there is one or n kinds of substrate donors in the preparation method, where n is an integer greater than or equal to 2. When there are n kinds of substrate donors, the preparation method includes a first substrate donor is linked with a first substrate receptor to form a second substrate receptor; a second substrate donor is then linked with the second substrate receptor to form a third substrate receptor, until an (n-1)th substrate donor is linked with an (n-1)th substrate receptor to form an nth substrate receptor; and an nth substrate donor is linked with the nth substrate receptor to form the non-natural RNA.

Further, the 5' end of the first substrate receptor is the phosphate group; the 5' end of the nth substrate donor is a 5' protecting group; the 5' ends of the second substrate donor to the (n-1)th substrate donor are hydroxyl; and the 3' ends of the second substrate receptor to the nth substrate receptor are hydroxyl.

Further, all 5' ends of a precursor of the second substrate receptor to a precursor of the nth substrate receptor are 5' protecting groups, and the 5' protecting group includes the hydroxyl. Before the second substrate receptor to the nth substrate receptor are linked with the corresponding substrate donors, the 5' protecting groups on the precursor of the second substrate receptor to the precursor of the nth substrate receptor are replaced with the phosphate groups. The 5' ends of the precursor of the second substrate receptor to the precursor of the nth substrate receptor are respectively modified to the phosphate groups by using a polynucleotide kinase, so as to obtain the substrate receptor with the 5' end being the phosphate group, and the polynucleotide kinase is deactivated.

Further, there is one or n kinds of substrate receptors in the preparation method, where n is an integer greater than or equal to 2. When there are n kinds of substrate receptors, the preparation method includes a first substrate donor is linked with a first substrate receptor to form a second substrate donor; a second substrate receptor is linked with the second substrate donor to form a third substrate donor, until an (n-1)th substrate receptor is linked with an (n-1)th substrate donor to form an nth substrate donor; and an nth substrate receptor is linked with the nth substrate donor to form the non-natural RNA.

Further, the 3' end of the substrate receptor is a protecting group; all 3' ends of a precursor of the second substrate donor to a precursor of the nth substrate donor, as well as a precursor of the non-natural RNA are protecting groups. Before the second substrate donor to the nth substrate donor are linked with the corresponding substrate receptors, the protecting groups are deprotected to obtain the substrate donor with the 3' end being the hydroxyl or the non-natural RNA.

Further, the non-natural nucleotide includes: a ribonucleotide having one or more of a ribose 2' modification, a ribose skeleton modification, a base modification, or a phosphate skeleton modification; the ribose 2' modification includes a 2'-methoxy modification, a 2'-fluoro modification, a 2'-methoxyethyl modification, a 2'-FANA modification, a locked nucleic acid modification, or a hexitol nucleic acid modification; the ribose skeleton modification includes replacing ribose in a nucleotide with ribuloNA, TNA, tPhoNA, or dXNA; the base modification includes a denitrified adenine C7 modification, a denitrified guanosine C7 modification, a cytosine C5 modification, or a uridine C5 modification; and the phosphate skeleton modification includes a PS modification.

Further, the substrate donor and/or the substrate receptor includes one or more non-natural nucleotides. The 3' end base of the substrate donor and/or the 5' end base of the substrate receptor is the non-natural nucleotide.

Further, the substrate donor and/or the substrate receptor is composed of the non-natural nucleotides.

The lengths of the substrate donor and the substrate receptor are both 2-500 nt.

Further, the length of the non-natural RNA is > 3 nt.

Further, the 3' end of the substrate receptor is the protecting group; and the 5' end of the substrate donor is the 5' protecting group.

Further, the protecting group includes an N-acetylgalactosamine group, a phosphate group, -O-NH2, azido, cyanoethyl, allyl, or 2-nitrobenzyl; and the 5' protecting group includes hydroxyl, hydrogen, or methyl.

According to a second aspect of the present disclosure, non-natural RNA is provided. The non-natural RNA includes the non-natural RNA that is obtained through preparation using the above preparation method.

By using the technical solutions of the present disclosure, in the above preparation method, non-natural RNA fragments are assembled through an RNA ligase catalyzed synthesis method, so as to synthesize a longer non-natural RNA strand, and a specific and efficient linkage may be realized without introduction of a template strand. Therefore, high-efficiency catalysis may be achieved, and efficiency and yield can be effectively improved compared to synthesis of non-natural RNA using a chemical method.

### Brief Description of the Drawings

The drawings, which form a part of the present application, are used to provide a further understanding of the present disclosure. The exemplary embodiments of the present disclosure and the description thereof are used to explain the present disclosure, but do not constitute improper limitations to the present disclosure. In the drawings:
Fig. 1 is a schematic diagram of preparation of non-natural RNA by connecting a plurality of substrate donors and substrate receptors according to an embodiment of the present disclosure.
Fig. 2 is a schematic diagram of preparation of non-natural RNA by connecting a plurality of substrate receptors and substrate donors according to an embodiment of the present disclosure.
Fig. 3 is a diagram of an SDS-PAGE gel electrophoresis result according to Embodiment 2 of the present disclosure.
Fig. 4 is a diagram of an SDS-PAGE gel electrophoresis result according to Embodiment 3 of the present disclosure.
Fig. 5 is a UPLC chromatogram according to Embodiment 3 of the present disclosure.
Fig. 6 is a UPLC chromatogram according to Embodiment 4 of the present disclosure.
Fig. 7 is a UPLC chromatogram according to Embodiment 4 of the present disclosure.
Fig. 8 is a schematic structural diagram of L96 according to Embodiment 4 of the present disclosure.
Fig. 9 is a schematic structural diagram of substrates 9, 10, and 11 according to Embodiment 5 of the present disclosure.

### Detailed Description of the Embodiments

It is to be noted that the embodiments in the present application and the features in the embodiments may be combined with one another without conflict. The present disclosure will be described below in detail with reference to the embodiments.

As mentioned in the Background, the application of using ligases to link single-stranded RNA is largely confined to natural RNA strands, and there are virtually no reports in the literature or patents regarding the enzymatic synthesis of non-natural single-stranded RNA. Therefore, the inventor of the present application attempts to develop a method for preparing non-natural RNA. Non-natural RNA fragments are assembled through an RNA ligase catalyzed synthesis method, so as to synthesize a longer non-natural RNA strand. Thus, a series of protective solutions of the present application are proposed.

In a first typical implementation of the present application, a method for preparing non-natural RNA is provided. The preparation method includes: the 3' end of a substrate donor is linked with the 5' end of a substrate receptor to form a phosphodiester bond by using a RNA ligase, where the linking does not require a template strand to prepare non-natural RNA; the substrate donor and/or the substrate receptor includes one or more non-natural nucleotides; the 5' end of the substrate receptor is a phosphate group; the 3' end of the substrate donor is a hydroxyl group; and the RNA ligase includes any one or more enzymes in the RNA ligase family Rnl1 and Rnl2.

In the above preparation method, a linkage can be completed by using the RNA ligase to connect the substrate donor and the substrate receptor without a template strand. The 5' end of the substrate donor is linked with the 3' end of the substrate receptor by using the phosphodiester bond. The 5' end of the substrate donor is the phosphate group, and the 3' end of the substrate receptor is the hydroxyl group.

There are one or more kinds of substrate receptors or substrate donors. When there are a plurality of substrate receptors, step-by-step linkage may be performed through the RNA ligase, and after the substrate receptor is linked with the substrate donor to form a new substrate donor, a new substrate receptor is linked with the new substrate donor. Similarly, when there are a plurality of substrate donors, step-by-step linkage may be performed through the RNA ligase, and after the substrate donor is linked with the substrate receptor to form the new substrate receptor, the new substrate donor is linked with the new substrate receptor. The linkage between the plurality of substrate receptors and substrate donors can be realized by using the above method, so as to realize preparation of long-strand non-natural RNA, thereby preventing an impact of linkage efficiency and RNA stability due to a too long RNA fragment.

In the present application, the concept of the substrate donor and substrate receptor does not restrict the specific nature of a substrate. Only the substrate located in a 5' direction during RNA linkage is defined as the substrate donor, and the substrate in a 3' direction is defined as the substrate receptor. In different reactions, the same reaction substrates may be the substrate donors, or may also be the substrate receptors.

In a preferred embodiment, the RNA ligase includes one or more of the RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 22; or an enzyme that has more than 70%, preferably, more than 75%, more than 80%, more than 85%, more than 90%, more than 95%, more than 98%, more than 99%, more than 99.5%, or more than 99.9% identity to any one of the RNA ligases shown in SEQ ID NO: 1-SEQ ID NO: 8 or SEQ ID NO: 15-SEQ ID NO: 22, and has catalytic activity for forming phosphodiester bonds.

The RNA ligase shown above is an RNA ligase that is screened in the present application and can link the non-natural RNA. The non-natural RNA includes a chemically modified nucleotide, which is artificially created and not natural. Therefore, the activity of natural enzymes toward non-natural nucleic acids remains unknown. In the present application, the above RNA ligase that can link the non-natural RNA without depending on the template strand is obtained by performing large-scale screening on current RNA ligases.

Identity in the present application refers to "identity" between amino acid sequences or nucleotide sequences, that is, the total of ratios of the amino acid residues or nucleotides of the same type in the amino acid sequences or nucleotide sequences. The identity of the amino acid sequences or nucleotide sequences may be determined by using comparison programs such as a Basic Local Alignment Search Tool (BLAST), FASTA, etc.

Active sites, active pockets, active mechanisms, protein structures, etc. of proteins having more than 70%, 75%, 80%, 85%, 90%, 95%, 99% (e.g., more than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even more than 99.9%) identity and having the same functions are probably the same as the protein provided the sequence in a).

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

For rules of substitution, replacement, etc., if amino acids are similar in nature, replacements have similar effects. For example, in the homologous protein, conservative amino acid replacements may occur. "Conservative amino acid replacement" includes, but is not limited to the followings.

Hydrophobic amino acids (Ala, Cys, Gly, Pro, Met, Val, Ile, and Leu) are substituted by other hydrophobic amino acids.

Hydrophobic amino acids with bulky side chains (Phe, Tyr, and Trp) are substituted by other hydrophobic amino acids with bulky side chains.

Amino acids with positively charged side chains (Arg, His, and Lys) are substituted by other amino acids with positively charged side chains.

Amino acids with polarized and uncharged side chains (Ser, Thr, Asn, and Gln) are substituted by other amino acids with polarized and uncharged side chains.

Person skilled in the art may also make conservative substitutions of the amino acids according to amino acid substitution rules known to person skilled in the art, such as a "blosum62 scoring matrix" in the prior art.

In a preferred embodiment, there is one or n kinds of substrate donors in the preparation method, where n is an integer greater than or equal to 2. When there are n kinds of substrate donors, the preparation method includes a first substrate donor is linked with a first substrate receptor to form a second substrate receptor; a second substrate donor is then linked with the second substrate receptor to form a third substrate receptor, until an (n-1)th substrate donor is linked with an (n-1)th substrate receptor to form an nth substrate receptor; and an nth substrate donor is linked with the nth substrate receptor to form the non-natural RNA.

In a preferred embodiment, the 5' end of the first substrate receptor is the phosphate group; the 5' end of the nth substrate donor is a 5' protecting group; the 5' ends of the second substrate donor to the (n-1)th substrate donor are hydroxyl; and the 3' ends of the second substrate receptor to the nth substrate receptor are hydroxyl.

In a preferred embodiment, all 5' ends of a precursor of the second substrate receptor to a precursor of the nth substrate receptor are 5' protecting groups, and the 5' protecting group includes, but is not limited to, the hydroxyl. Before the second substrate receptor to the nth substrate receptor are linked with the corresponding substrate donors, the 5' protecting groups on the precursor of the second substrate receptor to the precursor of the nth substrate receptor are replaced with the phosphate groups. The 5' ends of the precursor of the second substrate receptor to the the precursor of nth substrate receptor are respectively modified to the phosphate groups by using a polynucleotide kinase, so as to obtain the substrate receptor with the 5' end being the phosphate group, and the polynucleotide kinase is deactivated.

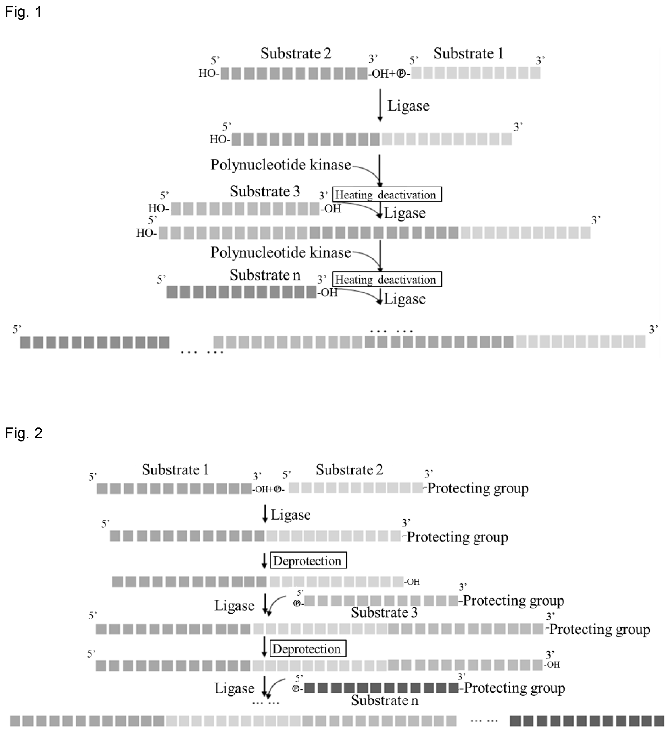

When there are n kinds of substrate donors above, it indicates that n kinds of substrate donors as substrate raw materials in the preparation method are gradually linked with the substrate receptor to form a new substrate receptor, and the new substrate receptor is further linked with a new substrate donor, so as to link the substrate receptor with the n kinds of substrate donors. When the plurality of substrate donors are linked with the substrate receptor using the above method, since the 5' end of a precursor of a newly-generated substrate receptor is not a phosphate group, polynucleotide kinase is required, the 5' end of the new substrate donor (i.e., a 5' end base of an original substrate receptor) formed by linking the substrate receptor with the substrate donor is modified as the phosphate group, and then the hydroxyl of the 3' end of the new substrate receptor is catalyzed by using the RNA ligase and linked with the phosphate group. Reaction steps are shown in Fig. 1. The substrate 1 in Fig. 1 is the above first substrate receptor, the substrate 2 is the above first substrate donor, and the substrate 3 is the above second substrate donor.

In a preferred embodiment, there is one or n kinds of substrate receptors in the above preparation method, where n is an integer greater than or equal to 2. When there are n kinds of substrate receptors, the preparation method includes the first substrate donor is linked with the first substrate receptor to form the second substrate donor; the second substrate receptor is linked with the second substrate donor to form a third substrate donor, until the (n-1)th substrate receptor is linked with the (n-1)th substrate donor to form the nth substrate donor; and the nth substrate receptor is linked with the nth substrate donor to form the non-natural RNA.

In the above preparation method, when there are n kinds of substrate receptors above, it indicates that n kinds of substrate receptors as substrate raw materials in the preparation method are gradually linked with the substrate donor to form a new substrate donor, and the new substrate donor is further linked with a new substrate receptor, so as to link the substrate donor with the n kinds of substrate receptors. Reaction steps are shown in Fig. 2. The substrate 1 in Fig. 2 is the above first substrate donor, the substrate 2 is the above first substrate receptor, and the substrate 3 is the above second substrate receptor.

In a preferred embodiment, the 3' end of the substrate receptor is a protecting group; all 3' ends of a precursor of the second substrate donor to a precursor of the nth substrate donor, as well as a precursor of the non-natural RNA are protecting groups. Before the second substrate donor to the nth substrate donor are linked with the corresponding substrate receptors, the protecting groups are deprotected to obtain the substrate donor with the 3' end being the hydroxyl or the non-natural RNA.

In order to prevent the self-linkage of the substrate receptors from affecting a reaction yield and product purity, the 3' end of the substrate receptor is the protecting group. After the substrate receptor is linked with the substrate donor to form the new substrate donor, the 3' end of the new substrate donor is a protecting group, and the new substrate donor needs to be deprotected to obtain the substrate donor with the 3' end being the hydroxyl, thereby realizing a further linkage with the new substrate receptor.

In a preferred embodiment, the non-natural nucleotide includes, but is not limited: a ribonucleotide having one or more of a ribose 2' modification, a ribose skeleton modification, a base modification, or a phosphate skeleton modification; preferably, the ribose 2' modification includes, but is not limited, a 2'-methoxy modification, a 2'-fluoro modification, a 2'-methoxyethyl modification, a 2'-FANA modification, a locked nucleic acid modification, or a hexitol nucleic acid modification; preferably, the ribose skeleton modification includes, but is not limited, replacing ribose in a nucleotide with ribuloNA, TNA, tPhoNA, or dXNA; preferably, the base modification includes, but is not limited, a denitrified adenine C7 modification, a denitrified guanosine C7 modification, a cytosine C5 modification, or a uridine C5 modification; and the phosphate skeleton modification includes a PS modification.

The locked nucleic acid modification is 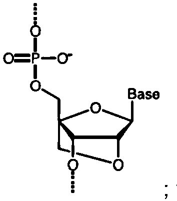 the hexitol nucleic acid modification is 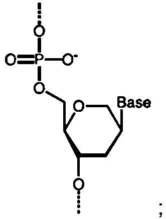 2'methoxyethyl modification is 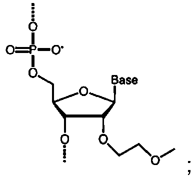 2'methoxy modification is 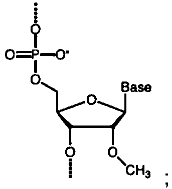 2'fluoro modification is 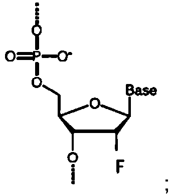 2'-FANA is 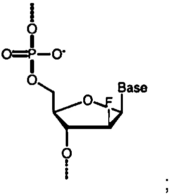 ribuloNA is 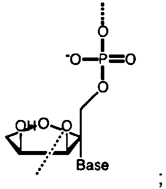 TNA is 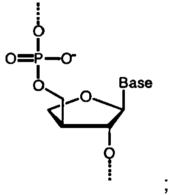 tPhoNA is 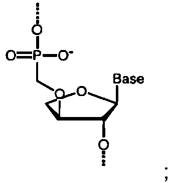 dXNA is 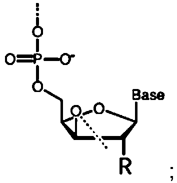 and PS modification is 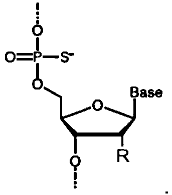 Bases in the above structures all represent bases, R represents a hydroxyl, hydrogen, methoxy, methoxyethyl, or fluoro modification. For the above modification of non-natural nucleotides, refer to literature: Duffy K, Arangundy-Franklin S, Holliger P. Modified nucleic acids: replication, evolution, and next-generation therapeutics[J]. BMC Biology, 2020, 18(1): 112.

In a preferred embodiment, the substrate donor and/or the substrate receptor includes one or more non-natural nucleotides. Preferably, the 3' end base of the substrate donor and/or the 5' end base of the substrate receptor is the non-natural nucleotide. Preferably, the substrate donor and/or the substrate receptor is composed of the non-natural nucleotides.

Xeno-nucleic acids (XNA) are a class of nucleic acid molecules having non-natural backbones or nucleic acid bases. Non-natural RNA is ribonucleotide having a non-natural backbone or nucleic acid base. The above substrate donor and/or substrate receptor contains the non-natural nucleotide, and the RNA prepared through linkage contains the non-natural nucleotide, belonging to the non-natural RNA. When the 3' end base of the substrate donor and/or the 5' end base of the substrate receptor are the non-natural nucleotide, although a base at a junction is different from a natural base and belongs to an artificially-created non-natural base, the above RNA ligase can also achieve linkage activity.

In a preferred embodiment, the lengths of the substrate donor and the substrate receptor are are 2-500 nt, including, but not limited to, 2, 3, 4, 5, 6, 7, 8, 9, 10, 15, 20, 25, 30, 40, 50, 60, 80, 100, 150, 200, 300, 400, and 500 nt.

In a preferred embodiment, the length of the non-natural RNA is >3 nt. With the non-natural RNA formed by linking the substrate donor and substrate receptor with the above length, one or more substrate receptors and substrate donors are linked, such that the non-natural RNA with the length > 3 nt can be obtained. The length of the above non-natural RNA includes, but is not limited to, 4, 5, 6, 7, 8, 9, 10, 12, 15, 20, 25, 30, 35, 40, 45, 50, 60, 70, 80, 90, 100, 200, 300, 400, 500, 600, 700, 800, 900, and 1000 nt.

In a preferred embodiment, the 3' end of the substrate receptor is the protecting group; preferably, the 5' end of the substrate donor is the 5' protecting group; preferably, the protecting group includes an N-acetylgalactosamine group (GalNac), a phosphate group, -O-NH₂, azido (-C-N = N⁺ = N⁻, 3'-O-azidomethyl), cyanoethyl (-C-CN, 3'-O-2-cyanoethyl), allyl (-C-C = C, 3'-O-allyl), or 2-nitrobenzyl (3'-O-2-nitrobenzyl); and preferably, the 5' protecting group includes hydroxyl, hydrogen, or methyl.

In a second typical implementation of the present application, non-natural RNA is provided. The non-natural RNA includes the non-natural RNA prepared according to the above preparation method.

Sequences, sources, and families of the ligases used in the present application are as follows.
Ligase 1, SEQ ID NO: 1, *Bacteriophage,* RNA ligase 2: 
MFKKYSSLENHYNSKFIEKLYSLGLTGGEWVAREKIHGTNFSLIIERDKVTCAKRTGPILPAEDF
FGYEIILKNYADSIKAVQDIMETSAVVSYQVFGEFAGPGIQKNVDYCDKDFYVFDIIVTTESGDVTYV
DDYMMESFCNTFKFKMAPLLGRGKFEELIKLPNDLDSVVQDYNFTVDHAGLVDANKCVWNAEAK
GEVFTAEGYVLKPCYPSWLRNGNRVAIKCKNSKFSEKKKSDKPIKAKVELSEADNKLVGILACYVTL
NRVNNVISKIGEIGPKDFGKVMGLTVQDILEETSREGITLTQADNPSLIKKELVKMVQDVLRPAWIEL
VS.
Ligase 2, SEQ ID NO: 2, *Vibrio phage,* RNA ligase 2: 
MSFVKYTSLENSYRQAFVDKCDMLGVREWVALEKIHGANFSFIVEFKPNEAQDGAEFTVTPA
KRTSTIGANVMGDYDFYGCTSVVEAHTAKMEAISNWLWARGIINVGETIIVYGELAGKGVQKEVNY
GDKDFWAYDILLPETGKFLDWDVVLEACEFAKVKTTHEIARGTLDELLRIDPLFRSFHTPADVDGDN
VAEGFVVKQLRNEKRLHNGSRAILKVKNDKFKEKKNKAGKTPRAAVVLTEEQEKLHAAFSCYLTEN
RLRNVLSKLETVTQKQFGMISGLFIKDAKDEFERDELNETAIARDDWDVIKRSLTNIANEILRKNWLD
ILDGNF.
Ligase 3, SEQ ID NO: 3, *Bacteriophage* AR1, RNA ligase 1: 
MQELFNNLMELCKDSQRKFFYSDDVSASGRTYRIFSYNYASYSDWLLPDALECRGIMFEMDG
EKPVRIASRPMEKFFNLNENPFTMNIDLNDVDYILTKEDGSLVSTYLDGDEILFKSKGSIKSEQALMA
NGILMNINHHQLRDRLKELAEDGFTANFEFVAPTNRIVLAYQEMKIILLNIRENETGEYISYDDIYKDA
ALRPYLVERYEIDSPKWVEEAKNAENIEGYVAVMKDGSHFKIKSDWYVSLHSTKSSLDNPEKLFKTI
 
IDGASDDLKAMYADDEYSYRKIEAFETTYLKYLDRALFLVLDCHNKHCGKDRKTYAMEAQGVAKGA
GMDHLFGIIMSLYQGYDSQEKVMCEIEQNFLKNYKKFIPEGY.
Ligase 4, SEQ ID NO: 4, *Bacteriophage dhaeg,* RNA ligase 2: 
MFKKYSSLENHYNSKFIEKLRTNGLTGGEWVAREKIHGTNFSLIIERDAVTCAKRTGPILPAEDF
YGYEIVLKNYADSIKSIQDIMETSAAVSYQVFGEFAGTGIQKNVDYGDKDFYVFDIIVTTESGDVTYV
DDYMMESFCKTFKFKMAPLLGRGKFEDLIKLPNDLDSVLPDYNFTVDNVGLAEANAHVWNAEAK
GEVFTAEGYVLKPCYPLWLPNGNRVAIKCKNSKFSEKKKTDKPIKVAVVLSQDDLDLLQQFTDYVT
VNRINNVISKIGEVSPKDFGKVMGLTVQDILEEAAREELELTDAENPVEVKKQLIECVKDTLRAVWIE
LVSK.
Ligase 5, SEQ ID NO: 5, *Klebsiella phage KP179,* RNA ligase 1: 
MLELYKNLMNLCESSEVAKFFYKDFTGPMDGKFRVFSYHYASYSEWLKPDALECRGIMFEMD
GDTPIRIASRPMEKFFNLNENPLTMGIDISDVEYIMDKADGSLVSSYVDDGYLYLKSKTSLYSDQAR
QASALLNSEEYSSLHQVILELTLDGYTVNMEFVSPNNRVVLAYQEPQLFVLNVRNNTTGEYIKYDD
LYANAKIRPYLINAYGISDPTTWVEGVRELEGVEGYIAVLNTGQRFKVKTEWYSALHHTKDSITSNE
RLFASVVSANSDDLRSLFAGDEYAIKKISAFEQAYLDYLGKSLELCQSFYDEYRGRARKDYAIAAQK
ATVNQRHLFGVIMNMYEGTVDVDKLLKDLERVFLKYWAGYVPKEYEKEIELSEE.
Ligase 6, SEQ ID NO: 6, *Vibrio phage,* RNA ligase 1: 
MTTQELYNHLMTLTDDAEGKFFFADHISPLGEKLRVFSYHIASYSDWLLPGALEARGIMFQLD
EQDKMVRIVSRPMEKFFNLNENPFTMDLDLTTTVQLMDKADGSLISTYLTGENFALKSKTSIFSEQA
VAANRYIKLPENRDLWEFCDDLTQAGCTVNMEWCAPNNRIVLEYPEAKLVILNIRDNETGDYVSFD
DIPLPALMRVKKWLVDEYDPETAHADDFVEKLRATKGIEGMILRLANGQSVKIKTQWYVDLHSQKD
SVNVPKKLVTTILNNNHDDLYALFADDKPTIDRIREFDSHVSKTVSASFHAVSQFYVKNRHMSRKDY
AIAGQKTLKPWEFGVAMIAYQNQTVEGVYEALVGAYLKRPELLIPEKYLNEA.
Ligase 7, SEQ ID NO: 7, *Escherichia phage* JN02, RNA ligase 1: 
MEKLYYNLLSLCKSSSDRKFFYSDDVSPIGKKYRIFSYNFASYSDWLLPDALECRGIMFEMDG
ETPVRIASRPMEKFFNLNENPFTLSINLDDVKYLMTKEDGSLVSTYLDGGTVRFKSKGSIKSDQAVS
ATSILLDIDHKNLADRLLELCNDGFTANFEYVAPTNKIVLTYPEKRLILLNIRDNNTGEYIEYDDIYLDP
VFRKYLVDRFEVPEGDWTSDVKSSTNIEGYVAVMKDGSHFKLKTDWYVALHTTRDSISSPEKLFLA
IVNGASDDLKAMYADDEFSFKKVELFEKAYLDFLDRSFYICLDTYDKHGKDRKTYAIEAQAVCKGA
QTPWLFGIIMNLYQGGSKEQMMTALESVFIKNHKNFIPEGY.
Ligase 8, SEQ ID NO: 8, *Klebsiella phage* KP15, RNA ligase 2: 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
EFLPDNLCSLISRSHGLKMAPLLGYGTFEEIKELPITFESVVNKANSGIGSDNTVYGEFVYPIMDVEE
 
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFIEQFEDPAMAKKLFVTEAQNMIRPV
WGKILNHEF.
Ligase 9, SEQ ID NO: 9, *Thermococcus,* RNA ligase 2-like: 
MVSSYFRNLLLKLGLPEERLEVLEGKGALAEDEFEGIRYVRFRDSARNFRRGTVVFETGEAVL
GFPHIKRVVQLENGIRRVFKNKPFYVEEKVDGYNVRVVKVDKILAITRGGFVCPFTTERIEDFVNF
DFFKDYPNLVLVGEMAGPESPYLVEGPPYVKEDIEFFLFDIQEKGTGRSLPAEERYRLAEEYGIPQV
ERFGLYDSSKVGELKELIEWLSEEKREGIVMKSPDMRRIAKYVTPYANINDIKIGSHIFFDLPHGYFM
GRIKRLAFYLAENHVRGEEFENYAKALGTALLRPFVESIHEVANGGEVDETFTVRVKNITTAHKMVT
HFERLGVKIHIEDIEDLGNGYWRITFKRVYPDATREIRELWNGLAFVD.
Ligase 10, SEQ ID NO: 10, *Archaea,* tRNA-splicing ligase: 
MVVPLKRIDKIRWEIPKFDKRMRVPGRVYADEVLLEKMKNDRTLEQATNVAMLPGIYKYSIVM
PDGHQGYGFPIGGVAAFDVKEGVISPGGIGYDINCGVRLIRTNLTEKEVRPRIKQLVDTLFKNVPSG
VGSQGRIKLHWTQIDDVLVDGAKWAVDNGYGWERDLERLEEGGRMEGADPEAVSQRAKQRGAP
QLGSLGSGNHFLEVQVVDKIFDPEVAKAYGLFEGQVVVMVHTGSRGLGHQVASDYLRIMERAIRK
YRIPWPDRELVSVPFQSEEGQRYFSAMKAAANFAWANRQMITHWVRESFQEVFKQDPEGDLGM
DIVYDVAHNIGKVEEHEVDGKRVKVIVHRKGATRAFPPGHEAVPRLYRDVGQPVLIPGSMGTASYIL
AGTEGAMKETFGSTCHGAGRVLSRKAATRQYRGDRIRQELLNRGIYVRAASMRVVAEEAPGAYK
NVDNVVKVVSEAGIAKLVARMRPIGVAKGAAALEH.
Ligase 11, SEQ ID NO: 11, *thermophilic bacteriophage,* RNA ligase 1: 
MESMNVKYPVEYLIEHLNSFESPEVAVESLRKEGIMCKNRGDLYMFKYHLGCKFDKIYHLACR
GAILRKTDSGWKVLSYPFDKFFNWGEELQPEIVNYYQTLRYASPLNEKRKAGFMFKLPMKLVEKL
DGTCVVLYYDEGWKIHTLGSIDANGSIVKNGMVTTHMDKTYRELFWETFEKKYPPYLLYHLNSSYC
YIFEMVHPDARVVVPYEEPNIILIGVRSVDPEKGYFEVGPSEEAVRIFNESGGKINLKLPAVLSQEQN
YTLFRANRLQELFEEVTPLFKSLRDGYEVVYEGFVAVQEIAPRVYYRTKIKHPVYLELHRIKTTITPE
KLADLFLENKLDDFVLTPDEQETVMKLKEIYTDMRNQLESSFDTIYKEISEQVSPEENPGEFRKRFA
LRLMDYHDKSWFFARLDGDEEKMQKSEKKLLTERIEKGLFK.
Ligase 12, SEQ ID NO: 12, *Diplonema papillatum,* RNA ligase 2: 
MPLSQGAALIRSPTVTAEEKVDGSNLAVYLDASGAVTCQNRGKFVTPSSGSQWGGQLATWL
ESHYCELVSLLRQRYILYGEWLLARHSIRYQSLPDYFVAFDVYDRTARRFLSAKKRNAFLSQSTIPV
VKPLAVGPFAEADILRLLGSPSRYGAAKVEGIYLRSDSGAWLQQRAKVVNSDFLQTIDDDGHWQK
RVLEKNQLKY.
Ligase 13, SEQ ID NO: 13, *virus,* RNA ligase 2-like: 
MVYICIDTGSHAKGYAVESSDTDYHIYTKCDRETFEKFIDNKELLKNRHAKDESGNDVKYVDLY
TGLIGILTGKSPELSMFSKREDFKDKYGIENLQLYEFVTKLMTVSMVKIIYTLMRYKILNNAKGLLQL
MFNYVYVEYYLDYKRAPKSTKILNMLFNVGDEIKITMDKNNQLVVNDLNVLDLNKNNGGYKIDETLT
LFVKNVKLLKLYVKLMQRGEYQQEWTEYFQQWKQQLQDRLHHVPEPPERTDIRHNIVMYALNER
GPVMPEDENKIVYQIYPSVSHLDQGKKGTLADKEIIVQEKLDGCNFRIICNQNKITYGSRNTYRPDG
NFMNYYRIRKDLETCMRSLQARFNDGFIVYGELMGWKDDAKTTPINVINYVDQKESLKYYAYEIQL
YGGEFVPFVEAQELLTNVGFNTIPCHKYLYNDFVERLNFKSLMFPQSPLEGFIIRCGNLIYKLKSDY
KDLNKLKIEKGPFEWLTCDYIKSNCDAIDKSDMMKILIFCYNMCKVKNYNEKLLFNKVFNLFRQQFN
LNHNDYKNLYKQYVNMCKCTEYK.
Ligase 14, SEQ ID NO: 14, *Archaea,* RNA ligase 3: 
MVSSVYKEILVKLGLTEDRIETLEMKGGIIEDEFDGIRYVRFKDSAGKLRRGTVVIDEEYVIPGF
PHIKRIINLRSGIRRIFKRGEFYVEEKVDGYNVRVVMYKGKMLGITRGGFICPFTTERIPDFVPQEFF
KDNPNLILVGEMAGPESPYLVEGPPYVKEDIQFFLFDVQEIKTGRSLPVEERLKIAEEYGINHVEVF
GKYTKDDVDELYQLIERLSKEGREGIIMKSPDMKKIVKYVTPYANINDIKIGARVFYELPPGYFTSRIS
RLAFYLAEKRIKGEEFERVAKELGSALLQPFVESIFDVEQEEDIHELFKVRVKRIETAYKMVTHFEKL
GLKIEIVDIEEIKDGWRITFKRLYPDATNEIRELIGGKAFVD.
Ligase 15, SEQ ID NO: 15, *Trypanosoma brucei gambiense,* RNA ligase 2: 
MLRCLGVRHFRRTPLLFVGGDGSIFERYTEIDNSNERRINALKGCGMFEDEWIATEKVHGANF
GIYSIEGEKMIRYAKRSGIMPPNEHFFGYHILIPELQRYVTSIREMLCEKQKKKLHVVLINGELFGGK
YDHPSVPKTRKTVMVAGKPRTISAVQTDSFPQYSPDLHFYAFDIKYKETEGGDYTTLVYDEAIELFQ
RVPGLLYARAVIRGPMSKVAAFDVERFVTTIPPLVGMGNYPLTGNWAEGLVVKHSRLGMAGFDPK
GPTVLKFKCTAFQEISTDRAQGPRVDEMRNVRRDSINRAGVQLPDLESIVQDPIQLEASKLLLNHV
CENRLKNVLSKIGTEPFEKEEMTPDQLATLLAKDAMKDFLKDTEPSIVNIPVLIRKDLTRYVIFESRRL
VCSQWKDILKRQSPDFSE.
Ligase 16, SEQ ID NO: 16, *Acidobacteria bacterium,* RNA ligase 1: 
MKQMYDNLKALTQKNDMFFSSIQTTPSNKPVEIFSYHIASYSDWLEPDAIECRGIMFDITDKEN
PVILSRPMEKFFNLGENPLALPEDVLPLVKTIEEKRDGSLISTYLDDGKLYTKSKGSLYSDQANDSN
RFLMRSENKDFKQALKTLAELGYTANMEYTSPKNQIVLKYEKQELIVLNIRNTETGDYLTLDDLAEL
DAENPEHTIYTHIAERFVDYEEVDGMSDEDVERIRDMKGIEGYVLVGQNQRVKLKTDWYVNLHRL
KDNINNNRRLVESVVESRTDDLRQLFEHDQGSIDKIKEFEQYVLSKIKSCLAEVRTVYERVKHLDRK
HYAINAQRYVDYTPLFSVIMRQFDDYNEENVVENIKQIALKNVDDFIPMHYK.
Ligase 17, SEQ ID NO: 17, *Klebsiella phage KP27,* RNA ligase 2: 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
 
EFLPDNLCSLISRSHGLKMAPLLGYGTFEEIKELPITFESVVNKANSGIGSDNTVYGEFVYPIMDVEE
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFLEQFEDPAMAKKLFVTEAQNMIRPV
WGKILNHEF.
Ligase 18, SEQ ID NO: 18, *Vibrio phage,* RNA ligase 2: 
MNVQELYKNLMSLADDAEGKFFFADHLSPLGEKFRVFSYHIASYSDWLLPGALEARGIMFQLD
DNDEMIRIVSRPMEKFFNLNENPFTMELDLTTTVQLMDKADGSLISTYLSGENFALKSKTSIFSEQA
VAANRYIKKPENRDLWEFCDDCTQAGLTVNMEWCAPNNRIVLEYPEAKLVILNIRDNETGDYVSFD
DIPQSALMRVKQWLVDEYDPATAHEPDFVEKLRDTKGIEGMILRLANGQSVKIKTQWYVDLHSQKD
SVNVPKKLVTTILNGNHDDLYALFADDKPTIERIREFDSHVTKTLTNSFNAVRQFYARNRHLARKDYA
IAGQKVLKPWEFGVAMIAYQKQTVEGVYESLVTAYLKRPELAIPEKYLNGV.
Ligase 19, SEQ ID NO: 19, *Vibrio phage JN02,* RNA ligase 2: 
MFKKYSSLENHYNSKFIEKLYTNGLTTGVWVAREKIHGTNFSLIIERDNVTCAKRTGPILPAEDF
YGYEIVLKKYDKAIKAVQEVMESISTSVPVSYQVFGEFAGGGIQKGVDYGEKDFYVFDIIINTESDDT
YYMSDYEMQDFCNTFGFKMAPMLGRGTFDSLIMIPNDLDSVLAAYNSTASEDLVEANNCVFDANVI
GDNTAEGYVLKPCFPKWLSNGTRVAIKCKNSKFSEKKKSDKPVKTQVPLTEIDKNLLDVLACYVTL
NRVNNVISKIGTVTPKDFGKVMGLTVQDILEETSREGIVLTSSDNPNLVKKELVRMVQDVLRPAWIE
LVS.
Ligase 20, SEQ ID NO: 20, *Vibrio phage JS98,* RNA ligase 2: 
MFKKYSSLENHYNSKFIEKLRTNGLTGGEWVAREKIHGTNFSLIIERDAVTCAKRTGPILPAEDF
YGYEIVLKNYADSIKSVQHLIESINYQSYQIYGELAGPGIQKNVDYGDKDFYVFDIRVTKEDGTESVL
TDTLMEAFCIIHKFKVAPCLATGSFEDLIKLPNDFDSVIPDYNFAVDNAGLTIANSTDFIPKVEGKVFT
AEGFVLKPDIPTWLPNGNRVAIKCKNSKFSEKKKSDKPIKAAVVLSQDDMDLMWQFTDYVTVNRIN
NVISKIGEVSKKDFGKVMGLTVQDILEEAAREELELTDAENPVEVKKQLVECVKDTLRAVWIELVS.
Ligase 21, SEQ ID NO: 21, *Vibrio phage nt-1,* RNA ligase 2: 
MNELYNNLMTLAESAEGKFFFADHLSPQGEKFRVFSYHIASYSDWLLPDALEARGIMFQLDEN
DEMIRIVSRPMEKFFNLGENPFTMDLDLTTTVQLMDKADGSLISTYLTGDNFALKSKTSIYSEQAVA
ANRYIKHVDNRDLWEFCDDCAAQNFTVNMEWCAPNNRIVLEYTEPKLIILNIRDNETGQYVSFDDIP
IGALTRIKKWLVDEYDPMTAHVDDFVETLKAKKGIEGMILRLASGQSVKIKTTWYVDLHAQKDSVNS
PKKLVTTILNKNHDDLYALFADDKPTIERIREFDDHVSKKVVESFHAVSQYYTKNRHLSRKDFAIAGQ
KALKPWEFGVAMIAYQNQTVEGVVTMLINAYLKRPELLIPEKYLSEV.
Ligase 22, SEQ ID NO: 22, *Vibrio phage phi-ST2,* RNA ligase 2:

MSFVKYTSLENSYRQAFVDKCDMLGVKEWVALEKIHGANFSFIVEFKPSTEEVPGEMSVTPA
KRTSTIGANAMGDYDFYGCTSVVEAHIEKMQDISNWLFANDFIKNDETIIVYGELAGKGIQKEVNYG

DKDFWAYDILCPETGEFLDWDVVLKACKFAGVKTTHEIARGTLDELLKIDPLFRSFHTPADVDSENV
AEGFVVKQLKAEKRLHNGSRAILKVKNEKFKEKKNKQGKTPRAKVVLTEEQEKLHAAFSCYLTENR
LRNVLSKIGKVEAKQFGMVSGLFVKDAKDEFERDERDEVAIPRDDWDVIKRSLVNVANEILRKNWL
NIVDGTF.

The beneficial effects of the present application are further described in detail below with reference to specific embodiments.

### Embodiment 1: Purification and preparation of RNA ligase

A gene encoding an RNA ligase was constructed on a pET28a(+) vector, and transformed into an E. coli BL21(DE3) expression strain. The expression strain was inoculated in a 5mL LB culture medium (containing 50 µg/mL) based on a 0.1% inoculation amount, and cultured for 16 h at 37 °C. A culture solution obtained was transferred into a 500mL LB culture medium based on a 1% inoculation amount, culture was performed at 37 °C until OD₆₀₀ = 0.6, 0.1 mM IPTG was added, and induction was performed for 16 h at 20 °C. An induced culture solution obtained was centrifuged to obtain bacterial sludge. The bacterial sludge was resuspended with a lysis buffer (50 mM Tris-Cl pH8.0, 0-500 mM NaCl, 10% glycerol) to cause a final concentration of the bacteria to be 10%; and ultrasonication was performed, and a crude enzyme solution was obtained through centrifugation. 10 mM of imidazole was added to the crude enzyme solution; an enzyme solution obtained through filtration using a 0.22 µm filter membrane was purified by using a nickel column; desalination and solution exchange was performed, and then secondary purification was performed with a strong anion column; and the obtained enzyme solution was subjected to desalination and solution exchange and then stored in 30% glycerol.

### Embodiment 2: Catalysis of non-template-dependent non-natural RNA linkage using RNA ligase

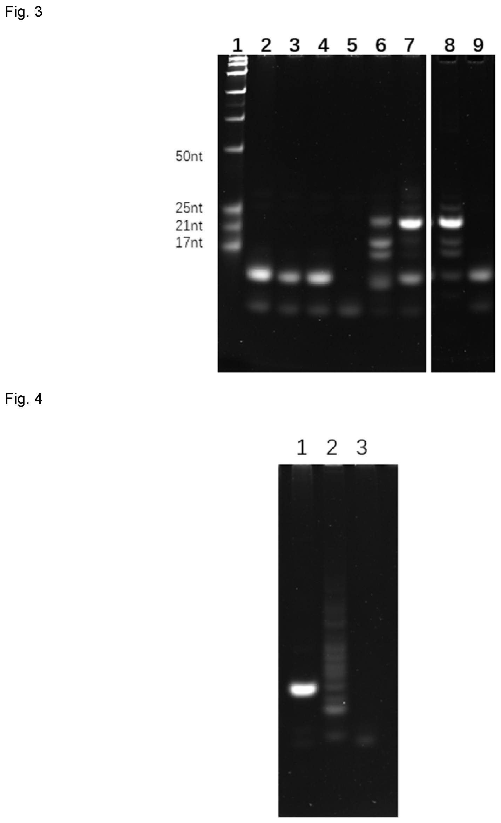

A substrate 1 and a substrate 2 were added to a clean reagent bottle (sequences of the substrate 1 and substrate 2 were shown in Table 1) to prepare a reaction system, causing a final volume to be 100 µL; and concentrations of the substrate 1 and substrate 2 were 500 µM, the concentration of ATP was 1 mM, the concentration of Tris-HCl (pH 7.5@25 °C) was 50 mM, the concentration of MgCl₂ was 10 mM, the concentration of dithiothreitol (DTT) was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm, and supernatant was detected through SDS-PAGE and UPLC. Results showed that ligases 6 and 8 could catalyze synthesis of a final product; an SDS-PAGE electropherogram was shown in Fig. 3. A lane 1 was Marker, a lane 2 was a control group (without ligases), a lane 3 was a system that is catalyzed using a ligase 9, a lane 4 was a system that is catalyzed using a ligase 10, a lane 5 was a system that is catalyzed using a ligase 11, a lane 6 was a system that is catalyzed using a ligase 13, a lane 7 was a system that is catalyzed using the ligase 6, a lane 8 was a system that is catalyzed using the ligase 8, and a lane 9 was a system that is catalyzed using a ligase 12. Results of UPLC showed that the substrate had already basically undergone a reaction completely and been converted completely. It indicated that the method for synthesizing non-natural RNA through catalysis using a ligase was successful.

**Table 1**

| Substrate | Sequence | 5' end | 3' end |
|---|---|---|---|
| Substrate 1 (SEQ ID NO: 23) | 5'UmCmCmUmUmAmGfUmCfAfGfUmAm3' | Hydroxyl | Hydroxyl |
| Substrate 2 (SEQ ID NO: 24) | 5'GmAmUmAmUmUmUmUmAmUm3' | Phosphate group | Phosphate group |

The m after A, C, G, or U represented 2' methoxy modification for the ribonucleotide, and the f represented 2' fluoro modification for the ribonucleotide.

The ribonucleotides at positions 1, 2, 3, 4, 5, 6, 8, 12, and 13 of the substrate 1 had 2' methoxy modification, and the ribonucleotides at positions 7, 9, 10, and 11 had 2' fluoro modification.

The ribonucleotides at positions 1, 2, 5, and 6 of the substrate 2 had 2' methoxy modification, and the ribonucleotides at positions 3 and 4 had 2' fluoro modification.

However, not all ligases could realize the linking of the non-natural RNA. When the reaction was performed based on the above same conditions, the ligases 9, 10, 11, and 12 did not have the activity, and the ligase 13 produced a large number of residual substrates and generated impurity bands.

### Embodiment 3: Catalysis of non-template-dependent short-strand non-natural RNA linkage using RNA ligase

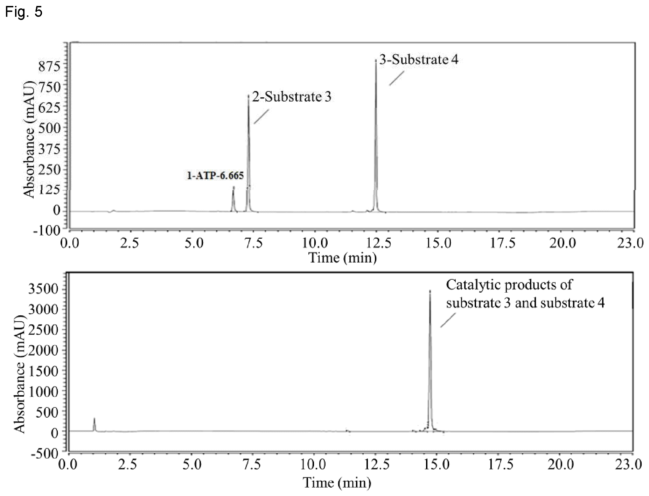

A substrate 3 and a substrate 4 were added to a clean reagent bottle (sequences of the substrate 3 and substrate 4 were shown in Table 2) to prepare a reaction system, causing a final volume to be 100 µL; and concentrations of the fragments 3 and 4 were 1000 µM, the concentration of ATP was 1.5 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. The obtained reaction system was held for 5 min at 80 °C to deactivate the ligase, a sediment was removed through centrifugation at 12000 rpm, and supernatant was detected through SDS-PAGE and UPLC. Results showed that in a control system (lane 3) without adding a ligase, a target product was not produced. The synthesis of a final product was found in a system that is added with the RNA ligase. An SDS-PAGE electropherogram was shown in Fig. 4. A lane 1 was a system that is catalyzed using a ligase 6, a lane 2 was a system that is catalyzed using a ligase 11, and a lane 3 was a control group (without ligase); and same screening was performed on other ligases. However, since the substrates 3 and 4 were relatively short, remaining substrates could not be observed clearly in SDS-PAGE, such that the reaction was detected using UPLC. Results showed that the substrate had already basically undergone a reaction completely and been converted completely. A UPLC chromatogram of the reaction catalyzed using a ligase 7 was shown in Fig. 5. It indicated that the method for synthesizing non-natural RNA through catalysis using a ligase was successful. RNA ligase used and catalytic activity were shown in Table 3.

**Table 2**

| Substrate | Sequence | 5' end | 3' end |
|---|---|---|---|
| Substrate 3 | 5'UmGmAfGfUmGm3' | Hydroxyl | Hydroxyl |
| Substrate 2 (SEQ ID NO: 25) | 5'GmAmCfUfGmCmAfCfUmAm3' | Phosphate group | Phosphate group |

The m after A, C, G, or U represented 2' methoxy modification for the ribonucleotide, and the f represented 2' fluoro modification for the ribonucleotide.

The ribonucleotides at positions 1, 2, 5, and 6 of the substrate 3 had 2' methoxy modification, and the RNA at positions 3 and 4 had 2' fluoro modification.

The ribonucleotides at positions 1, 2, 5, 6, 9, and 10 of the substrate 4 had 2' methoxy modification, and the RNA at positions 3, 4, 7, and 8 had 2' fluoro modification.

**Table 3**

| Enzyme | Source organism | Activity |
|---|---|---|
| Ligase 3 | *Bacteriophage* AR1 | +++ |
| Ligase 6 | *Vibrio phage* | +++ |
| Ligase 7 | *Escherichia phage* JN02 | +++ |
| Ligase 2 | *Vibrio phage* | +++ |
| Ligase 4 | *Bacteriophage dhaeg* | +++ |
| Ligase 5 | *Klebsiella phage KP179* | +++ |
| Ligase 1 | *Vibrio phage* | +++ |
| Ligase 19 | *Vibrio phage JN02* | ++ |
| Ligase 20 | *Vibrio phage JS98* | ++ |
| Ligase 21 | *Vibrio phage nt-1 rnlA* | ++ |
| Ligase 22 | *Vibrio phage phi-ST2* | ++ |
| Ligase 11 | *thermophilic bacteriophage* | - |
| Ligase 12 | *Diplonema papillatum* | - |
| Ligase 13 | *virus* | - |
| Ligase 14 | *Archaea* | - |
| Ligase 15 | *Trypanosoma brucei gambiense* | ++ |
| Ligase 16 | *Acidobacteria bacterium* | ++ |

| | | |
|---|---|---|
| Note: "+++" in the present application indicated that catalytic activity was 70%-100%, "++" indicated that catalytic activity was 60%-70% (not including an endpoint value of 70%), and "-" indicated that there was essentially no catalytic activity. | | |

### Embodiment 4: Catalysis of non-template-dependent long-strand non-natural RNA linkage using RNA ligase

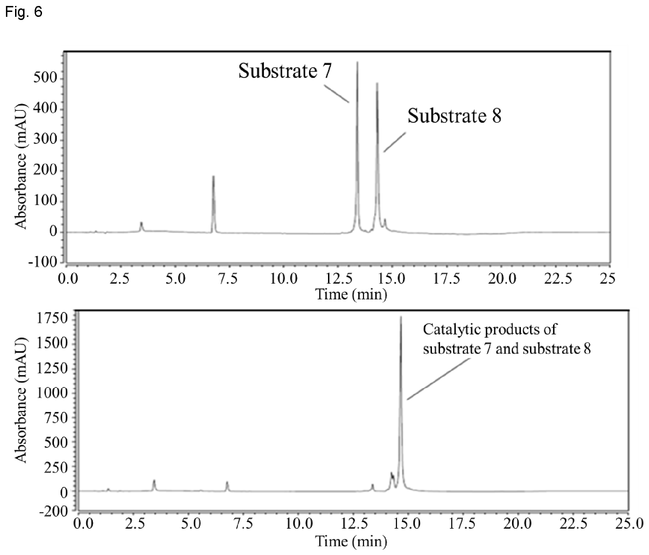

A substrate 7 and a substrate 8 were added to a clean reagent bottle to prepare a reaction system, causing a final volume to be 100 µL; and concentrations of the substrates 7 and 8 were 300 µM, the concentration of ATP was 0.6 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 37 °C. The obtained reaction system was held for 5 min at 80 °C to deactivate the ligase, a sediment was removed through centrifugation at 12000 rpm, and supernatant was detected through SDS-PAGE and UPLC. A UPLC chromatogram of the reaction catalyzed using a ligase 4 was shown in Fig. 6. Results showed that a product was produced in the system that is added with the RNA ligase. It indicated that the method for catalytic synthesis of non-natural RNA using a ligase was successful. RNA ligase used and catalytic activity were shown in Table 4.

**Table 4**

| Enzyme | Source organism | Activity |
|---|---|---|
| Ligase 2 | *Vibrio phage* | +++ |
| Ligase 4 | *Bacteriophage dhaeg* | +++ |
| Ligase 5 | *Klebsiella phage KP179* | +++ |
| Ligase 15 | *Trypanosoma brucei gambiense* | ++ |
| Ligase 16 | *Acidobacteria bacterium* | ++ |
| Ligase 17 | *Klebsiella phage KP27* | ++ |
| Ligase 18 | *Vibrio phage* | ++ |
| Ligase 19 | *Vibrio phage JN02* | ++ |
| Ligase 20 | *Vibrio phage JS98* | ++ |
| Ligase 21 | *Vibrio phage nt-1 rnlA* | ++ |
| Ligase 22 | *Vibrio phage phi-ST2* | ++ |

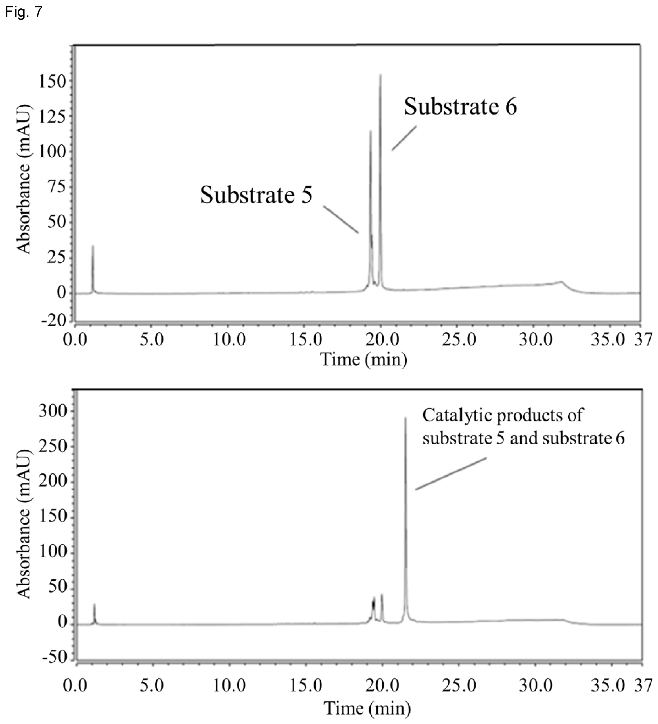

A substrate 5 and a substrate 6 were added to a clean reagent bottle to prepare a reaction system, causing a final volume to be 100 µL; and concentrations of the substrates 5 and 6 were 100 µM, the concentration of ATP was 0.2 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 37 °C. The obtained reaction system was held for 5 min at 80 °C to deactivate the ligase, a sediment was removed through centrifugation at 12000 rpm, and supernatant was detected through SDS-PAGE and UPLC. A UPLC chromatogram of the reaction catalyzed using a ligase 5 was shown in Fig. 7. Results showed that a final product was produced in the system that is added with the RNA ligase. It indicated that the method for catalytic synthesis of non-natural RNA using a ligase was successful. RNA ligase used and catalytic activity were shown in Table 5.

Sequences of substrates 5, 6, 7, and 8 were shown in Table 6.

**Table 5**

| Enzyme | Source organism | Activity |
|---|---|---|
| Ligase 1 | *Bacteriophage* | +++ |
| Ligase 2 | *Vibrio phage* | ++ |
| Ligase 4 | *Bacteriophage dhaeg* | +++ |
| Ligase 5 | *Klebsiella phage KP179* | +++ |
| Ligase 8 | *Klebsiella phage KP15* | +++ |
| Ligase 15 | *Trypanosoma brucei gambiense* | ++ |
| Ligase 16 | *Acidobacteria bacterium* | ++ |
| Ligase 17 | *Klebsiella phage KP27* | ++ |
| Ligase 18 | *Vibrio phage* | ++ |
| Ligase 19 | *Vibrio phage JN02* | ++ |
| Ligase 20 | *Vibrio phage JS98* | ++ |
| Ligase 21 | *Vibrio phage nt-1 rnlA* | ++ |

**Table 6**

| Serial number | Sequence | Size | 5' end | 3' end |
|---|---|---|---|---|
| Substrate 5 (SEQ ID NO: 26) | 5’GmAmCmUmGmAmUmAmUmAmCmAmCmAm GmGmCmCmGmAmUmGmUmGmGmUmUmUm UmAmGmAmGmCmUmAmGmAmAmAmUmAmG mCmAmAmGmUmUmAm3’ | 50nt | Hydroxyl | Hydroxyl |
| Substrate 6 (SEQ ID NO: 27) | 5’AmAmAmUmAmAmGmGmCmUmAmGmUmCm CmGmUmUmAmUmCmAmAmCmUmUmGmAmA mAmAmAmGmUmGmGmCmAmCmCmGmAmGm UmCmGmGmUmGmCm3’ | 50nt | Phosphate group | L96 |
| Substrate 7 (SEQ ID NO: 28) | 5’GmUmUmUmUmAmGmAmGmCmUmAmGmAm AmAmUmAmGmCmAmAmGmUmUmAmAmAmA mUmAmAmGmGmCmUm3’ | 36nt | Hydroxyl | Hydroxyl |
| Substrate 8 (SEQ ID NO: 29) | 5'AmGmUmCmCmGmUmUmAmUmCmAmAmCm UmUmGmAmAmAmAmAmGmUmGmGmCmAmC mCmGmAmGmUmCmGmGmUmGmCm3' | 40nt | Phosphate group | L96 |

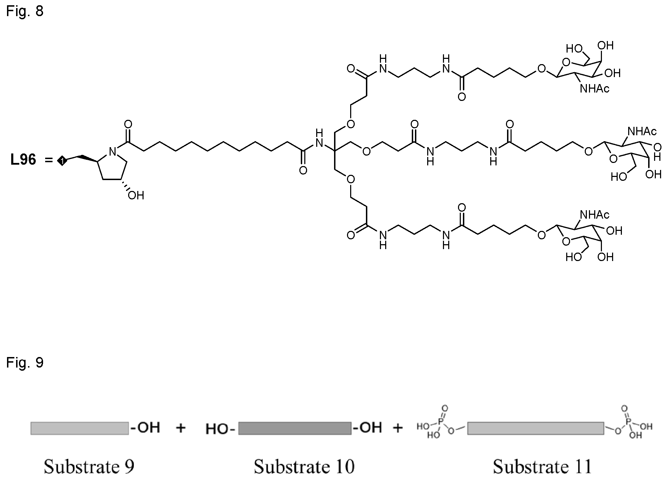

The structure of L96 was shown in Fig. 8, where 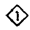 represented a base linked with an L96 group; and the m after A, C, G, or U represented 2' methoxy modification for the ribonucleotide.

The ribonucleotides of the substrates 5, 6, 7, and 8 all had 2' methoxy modification.

### Embodiment 5: Catalytic synthesis of multi-fragment non-natural RNA substrate linkage using RNA ligase

At Step 1: a substrate 10 and a substrate 11 were added to a clean reagent bottle to prepare a reaction system, causing a final volume to be 100 µL; and concentrations of the substrates 10 and 11 were 800 µM, the concentration of ATP was 1.6 mM, the concentration of Tris-HCl (pH 7.5@25 °C) was 50 mM, the concentration of MgCl₂ was 10 mM, the concentration of DTT was 1 mM, and the concentration of a ligase was 0.2 mg/mL. The reaction system underwent a reaction for 16 h at 16 °C. Through detection, the substrate had consumed completed.

At Step 2: 8 µL of 10U/µL T4 polynucleotide kinase was added, the reaction system underwent the reaction for 6 h at 37 °C, and the obtained reaction system was held for 5 min at 80 °C to deactivate the T4 polynucleotide kinase and the ligase.

At Step 3: a substrate 9 with an equal molar amount relative to the substrate 10 was added, the ATP with equal molar was additionally added, the concentration of a final system substrate was 200 µM, a 0.2mg/mL ligase was added, and a reaction was performed for 16 h at 16 °C. The obtained final reaction system was held for 5 min at 80 °C to deactivate the ligase, a sediment was removed through centrifugation at 12000 rpm, and supernatant was detected through SDS-PAGE and UPLC. Results showed that a final product was produced in the final system. The final product was formed by linking the substrate 9, substrate 10, and substrate 11 from the 5' end to the 3' end, and a conversion rate of the product reached more than 60%, indicating that the method for catalytic synthesis of non-natural RNA using an RNA ligase was successful. RNA ligase used and catalytic activity were shown in Table 7.

Sequences of the substrates 9, 10, and 11 were shown in Fig. 8, and schematic diagrams of the substrates 9, 10, and 11 were shown in Fig. 9.

**Table 7**

| | Ligase | Source organism | Activity |
|---|---|---|---|
| Step 1 | Ligase 2 | *Vibrio phage* | +++ |
| | Ligase 4 | *Bacteriophage dhaeg* | +++ |
| | Ligase 5 | *Klebsiella phage KP179* | +++ |
| | Ligase 17 | *Klebsiella phage KP27* | ++ |
| | Ligase 18 | *Vibrio phage* | ++ |
| | Ligase 19 | *Vibrio phage JN02* | ++ |
| Step 3 | Ligase 3 | *Bacteriophage AR1* | +++ |
| | Ligase 6 | *Vibrio phage* | +++ |
| | Ligase 7 | *Escherichia phage JN02* | ++ |
| | Ligase 20 | *Vibrio phage JS98* | ++ |
| | Ligase 21 | *Vibrio phage nt-1 rnlA* | ++ |

**Table 8**

| Serial number | Sequence | 5' end | 3' end |
|---|---|---|---|
| Substrate 9 | 5'UmGmAfGfUmGm3' | Hydroxyl | Hydroxyl |
| Substrate 10 | 5'AfGfAmCmCfUfGmCm3' | Hydroxyl | Hydroxyl |
| Substrate (SEQ ID NO: 30) | 5'GmAmCfUfGmCmAfCfUmAm3' | Phosphate group | Phosphate group |

The m after A, C, G, or U represented 2' methoxy modification for the ribonucleotide, and the f represented 2' fluoro modification for the ribonucleotide.

The ribonucleotides at positions 1, 2, 5, and 6 of the substrate 9 had 2' methoxy modification, and the ribonucleotides at positions 3 and 4 had 2' fluoro modification.

The ribonucleotides at positions 3, 4, 7, and 8 of the substrate 10 had 2' methoxy modification, and the ribonucleotides at positions 1, 2, 5 and 6 had 2' fluoro modification.

The ribonucleotides at positions 1, 2, 5, 6, 9, and 10 of the substrate 11 had 2' methoxy modification, and the ribonucleotides at positions 3, 4, 7, and 8 had 2' fluoro modification.

From the above description, it might be seen that the above embodiments of the present disclosure achieved the following technical effects. In the present disclosure, it was found that a portion of the RNA ligases could link non-natural nucleotides. By using the above preparation method, the above RNA ligase catalyzed synthesis method could link and assemble two or more non-natural RNA fragments, so as to synthesize a longer non-natural RNA strand, and a specific and efficient linkage may be realized without introduction of a template strand. Therefore, high-efficiency catalysis may be achieved, and efficiency and yield can be effectively improved compared to synthesis of non-natural RNA using a chemical method. Furthermore, the problem in the prior art of difficult usage of current RNA ligases for linking natural nucleic acids to link non-natural nucleotides was solved.

The above are only the preferred embodiments of the present disclosure and are not intended to limit the present disclosure. For person skilled in the art, the present disclosure may have various modifications and variations. Any modifications, equivalent replacements, improvements and the like made within the spirit and principle of the present disclosure all fall within the scope of protection of the present disclosure.

## Claims

1. A preparation method of a non-natural RNA, wherein the preparation method comprises:
linking the 3' end of a substrate donor with the 5' end of a substrate receptor to form a phosphodiester bond with a RNA ligase, wherein the linking does not require a template strand to prepare the non-natural RNA,
the substrate donor and/or the substrate receptor comprises one or more non-natural nucleotides;
the 5' end of the substrate receptor is a phosphate group;
the 3' end of the substrate donor is a hydroxyl group; and
the RNA ligase comprises any one or more enzymes in the RNA ligase family Rnl1 and Rnl2.

2. The preparation method according to claim 1, wherein the RNA ligase comprises:
one or more of the RNA ligases as shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22; or
an enzyme sharing more than 70% identity with any RNA ligase as shown in SEQ ID NO: 1 to SEQ ID NO: 8, or SEQ ID NO: 15 to SEQ ID NO: 22, and having an activity for catalyzing the formation of the phosphatediester bond.

3. The preparation method according to claim 1 or 2, wherein the preparation method comprises one or n kinds of substrate donors, wherein n is an integer greater than or equal to 2,
when the number of types of the substrate donors is n, the preparation method comprises:
linking a first substrate donor with a first substrate receptor to form a second substrate receptor;
then linking a second substrate donor with the second substrate receptor to form a third substrate receptor;
up until linking an (n-1)th substrate donor with an (n-1)th substrate receptor to form an nth substrate receptor; and
linking an nth substrate donor with the nth substrate receptor and forming the non-natural RNA.

4. The preparation method according to claim 3, wherein
the 5' end of the first substrate receptor is the phosphate group;
the 5' end of the nth substrate donor is a 5' protecting group;
the 5' ends of the second substrate donor to the (n-1)th substrate donor are hydroxyl groups; and
the 3' ends of the second substrate donor to the nth substrate donor are hydroxyl groups.

5. The preparation method according to claim 4, wherein all 5 'ends of a precursor of the second substrate receptor to a precursor of the nth substrate receptor are 5' protecting groups, and the 5' protecting groups comprise hydroxyl groups;
before linking the second substrate receptor to the nth substrate receptor with the corresponding substrate donor,
the 5' protecting groups on the precursor of the second substrate receptor to the precursor of the nth substrate receptor are replaced with the phosphate groups; and
the 5' ends of the precursor of the second substrate receptor to the precursor of the nth substrate receptor are modified to the phosphate group using a polynucleotide kinase, respectively to obtain the substrate receptor of which the 5' end is the phosphate group, and the polynucleotide kinase is deactivated.

6. The preparation method according to claim 1 or 2, wherein the preparation method comprises one or n kinds of substrate receptors, wherein n is an integer greater than or equal to 2;
when the number of types of the substrate receptors is n, the preparation method comprises:
linking a first substrate donor with a first substrate receptor to form a second substrate donor;
linking a second substrate receptor with the second substrate donor to form a third substrate donor;
up until linking an (n-1)th substrate receptor with an (n-1)th substrate donor to form an nth substrate donor; and
linking an nth substrate receptor with the nth substrate donor and forming the non-natural RNA.

7. The preparation method according to claim 6, wherein the 3' end of the substrate receptor is a protecting group; correspondingly, the 3' ends of the precursor of the second substrate donor to the precursor of the nth substrate donor which are obtained by linking are all the protecting group;
before linking the second substrate donor to the nth substrate donor with the corresponding substrate receptor, the protecting group is deprotected ,and the non-natural RNA or the substrate donor whose 3' end is a hydroxyl group, is obtained.

8. The preparation method according to claim 1, wherein the non-natural nucleotide comprises a ribonucleotide having one or more of a ribose position 2' modification, a ribose skeleton modification, a base modification or a phosphate skeleton modification;
the ribose position a 2' modification comprises a 2'-methoxy modification, a 2'-fluoro modification, a 2'-methoxyethyl modification, a 2'-FANA modification, a locked nucleic acid modification or a hexitol nucleic acid modification;
the ribose skeleton modification comprises replacing the ribose in the nucleotide with ribuloNA, TNA, tPhoNA or dXNA;
the base modification comprises a denitrified adenine C7 modification, a denitrified guanosine C7 modification, a cytosine C5 modification, or a uridine C5 modification; and
the phosphate skeleton modification comprises a PS modification.

9. The preparation method according to claim 1, wherein the substrate donor and/or the substrate receptor contains one or more of the non-natural nucleotides; and
the 3' end base of the substrate donor and/or the 5' end base of the substrate receptor is the non-natural nucleotide.

10. The preparation method according to claim 9, wherein the substrate donor and/or the substrate receptor is composed of the non-natural nucleotides.

11. The preparation method according to claim 1, wherein the length of both the substrate donor and the substrate receptor is 2-500 nt.

12. The preparation method according to claim 1, wherein the length of the non-natural RNA is > 3 nt.

13. The preparation method according to claim 1, wherein the 3' end of the substrate receptor is a protecting group; and the 5' end of the substrate donor is a 5' protecting group.

14. The preparation method according to claim 13, wherein the protecting group comprises an N-acetyl galactosamine group, a phosphate group, -O-NH₂, an azido group, a cyanoethyl group, an allyl group or a 2-nitrobenzyl group; and the 5' protecting group comprises a hydroxyl group, a hydrogen group, or a methyl group.

15. A non-natural RNA, wherein the non-natural RNA comprises the non-natural RNA obtained with the preparation method of any one of claims 1 to 14.
